(19) Europäisches Patentamt
European Patent Office
Office européen des brevets

(11) EP 2 158 954 A1

(12) EUROPEAN PATENT APPLICATION
published in accordance with Art. 153(4) EPC

(43) Date of publication:
03.03.2010 Bulletin 2010/09

(51) Int Cl.:
B01D 1/14 (2006.01)    C07C 45/38 (2006.01)
C07C 47/22 (2006.01)    C07C 51/235 (2006.01)
C07C 57/055 (2006.01)

(21) Application number: 08752785.9

(22) Date of filing: 15.05.2008

(86) International application number:
PCT/JP2008/058925

(87) International publication number:
WO 2008/143126 (27.11.2008 Gazette 2008/48)

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 15.05.2007   JP 2007129038

(71) Applicant: Mitsubishi Rayon Co. Ltd.
Minato-ku
Tokyo 108-8506 (JP)

(72) Inventors:
• SATOU, Toshihiro
Otake-shi
Hiroshima 739-0693 (JP)

• NISHIJIMA, Katsumasa
Otake-shi
Hiroshima 739-0693 (JP)
• MOMODOMI, Nobuo
Otake-shi
Hiroshima 739-0693 (JP)
• KURODA, Toru
Otake-shi
Hiroshima 739-0693 (JP)

(74) Representative: HOFFMANN EITLE
Patent- und Rechtsanwälte
Arabellastrasse 4
81925 München (DE)

(54) EVAPORATION APPARATUS, METHOD OF EVAPORATION AND PROCESS FOR PRODUCING METHACROLEIN OR (METH)ACRYLIC ACID

(57) Disclosed is an evaporation device evaporating part of a liquid raw material that comprises two or more components and that is used in gas-phase catalytic reaction accompanied with heat generation and supplying the part of the liquid raw material to a reactor for the gas-phase catalytic reaction accompanied with heat generation, which contains an evaporation section which is equipped with an evaporator having a vapor take-out port at the top and evaporating the liquid raw material by heating the liquid raw material from inside or outside or both inside and outside the evaporator; a column section composed of at least one of a plate column and a column containing packings, the bottom of which is connected to the vapor take-out port of the evaporator of the evaporation section, a liquid supply pipe supplying the liquid raw material to a middle or upper position of the column section, a vapor lead-out pipe leading out vapor from the top of the column section and supplying the vapor to the reactor of the gas-phase catalytic reaction, and a liquid discharge pipe discharging the liquid raw material in the evaporator.

FIG. 4

## Description

TECHNICAL FIELD

The present invention relates to an evaporation device and a method for evaporation that evaporates part of a liquid raw material that comprises two or more components and that is used in gas-phase catalytic reaction accompanied with heat generation, and a method for producing methacrolein or (meth)acrylic acid using the method for evaporation.
The present application claims the priority of Japanese Patent Application No. 2007-129,038 filed at the Japan Patent Office on May 15, 2007, the contents of which are hereby incorporated herein by reference.

BACKGROUND ART

In the petrochemical field, useful compounds are produced by reacting organic compounds with oxygen through gas-phase catalytic reactions. The gas-phase catalytic reaction is generally carried out by supplying a feed gas containing an organic compound which is a raw material to be oxidized and oxygen to a reactor for the gas-phase catalytic reaction. As the reactor for the gas-phase catalytic reaction, there are a variety of types depending on holding methods of a catalyst layer, however, a tubular reactor equipped with a reaction tube in which the catalyst layer is formed is popular and well known.

The feed gas to be supplied to the reactor for the gas-phase catalytic reaction is generally supplied from an evaporation device in which a liquid containing an organic compound which is a raw material to be oxidized (liquid raw material) is evaporated. As the evaporation device, an evaporator type evaporation device is popular, which is composed of an evaporator to hold the liquid and a heating device to supply heat quantity to the liquid inside the evaporator through a heating medium, and for example, a multitubular heating method in which a multitubular heat exchanger is connected to the evaporator and the liquid is heated with the multitubular heat exchanger, a vessel with an outer jacket method in which a jacket for heating is provided outside the evaporator and the evaporator is heated with the jacket, and a trace piping method in which a trace piping is provided outside the evaporator and the evaporator is heated with the trace piping can be listed (for example, refer to non-Patent Document 1).

Generally, in gas-phase catalytic reaction, an excellent reaction result is obtained by selecting an optimum catalyst and optimum values of reaction conditions such as composition of chemical components to be supplied to the reaction, reaction temperature, and reaction pressure. However, when a variation is caused in a feed rate or a composition of a feed gas, the reaction condition is shifted from an optimum condition, and hence the reaction result is deteriorated.

In particular, in the case of gas-phase catalytic reaction accompanied with heat generation, a variation in a heat of reaction immediately affects the reaction temperature. Consequently, the reaction condition is shifted from the optimum condition and the reaction result is deteriorated. Further, when the gas-phase catalytic reaction accompanied with heat generation is carried out with the tubular reactor, heat balance of the reaction is maintained by removing the heat quantity generated in the reaction tube with a heating medium outside the reaction tube, however, when the variation in the reaction condition as mentioned above is caused, the heat balance is changed, and the heat quantity exponentially accumulates in the catalyst layer, and thereby the reaction temperature is further raised and the generated heat quantity is increased. Under these circumstances, the gas-phase catalytic reaction changes into what is called the runaway reaction by the variation in the heat of reaction, and it is apprehended that seizing of the catalyst or, in extreme cases, breakage of the reaction tube may occur.

When the gas-phase catalytic reaction is carried out with the tubular reactor, the time for the feed gas to pass through the reaction tube of the tubular reactor (residence time) is generally from about 0.1 second to about several seconds. Consequently, the variation in the feed rate of the feed gas that becomes a problem in the gas-phase catalytic reaction is the one to be caused in such a short cycle as from one tenth of the residence time to several tens of times as large as the residence time. Therefore, it is important to suppress the variation in the feed rate or the composition of the feed gas in such a short cycle as about the aforementioned residence time to stably carry out the gas-phase catalytic reaction accompanied with heat generation.

The suppress of the variation in the feed rate of the feed gas from the evaporator type evaporation device to the reactor can be attained by suppressing variation in the feed rate of the feed gas to the evaporator and variation in the heat quantity to be supplied from the heating medium in the case that the substantially whole quantity of the liquid raw material is evaporated. The feed rate of the liquid raw material and the heat quantity to be supplied can be controlled by publicly known methods, and can be controlled, for example, by controlling temperature and flow rate of the heating medium.

However, when the liquid raw material contains two or more components that are supplied to the reactor after being evaporated, variation in the vapor composition is liable to occur in the case that part of the liquid raw material is evaporated (part of the liquid raw material is not evaporated) because the volatilities of these components are usually different. Among other things, when there are great differences in volatility between the components to be supplied to the reactor after being evaporated and the components to be left behind without being evaporated (impurities and the like) in the components contained in the liquid raw material, a liquid composition and a gas composition are greatly different, and the gas composition considerably varies with disturbance.

Consequently, there has been a desire of an evaporation device and a method for evaporation that can stably evaporate part of the liquid raw material that contains two or more components and that is used in the gas-phase catalytic reaction accompanied with heat generation so as not to cause variation in the feed rate to the reactor of the gas-phase catalytic reaction or the composition of the feed gas in such a short cycle as about the aforementioned residence time.

Non-Patent Document 1: Outline of Process Design, pp. 63-72, Chemical Engineering Society, published on August, 1973

## DISCLOSURE OF INVENTION PROBLEM TO BE SOLVED BY THE INVENTION

It is a subject of the present invention to provide an evaporation device and a method for evaporation that can stably evaporate part of a liquid raw material that contains two or more components and that is used in gas-phase catalytic reaction accompanied with heat generation, and a method for producing methacrolein or (meth)acrylic acid using the method for evaporation.

## MEANS FOR SOLVING THE PROBLEM

The present invention that solves the above-mentioned subject has the following modes.

(1) An evaporation device that evaporates part of a liquid raw material that comprises two or more components and that is used in gas-phase catalytic reaction accompanied with heat generation and supplies the part of the liquid raw material to a reactor for the gas-phase catalytic reaction, the evaporation device comprising: an evaporation section that is equipped with an evaporator having a vapor take-out port at the top and evaporates the liquid raw material by heating the liquid raw material from inside or outside or both inside and outside the evaporator; a column section composed of at least one of a plate column and a column containing packings, the bottom of which is connected to the vapor take-out port; a liquid supply pipe supplying the liquid raw material to a middle or upper position of the column section; a vapor lead-out pipe leading out vapor from the top of the column section and supplying the vapor to the reactor for the gas-phase catalytic reaction; and a liquid discharge pipe discharging the liquid raw material in the evaporator.

(2) The evaporation device according to (1), wherein the column section is composed of a plate column having a plate number of 1 to 10 or a packed column having a packed length equivalent to theoretical plate number of 0.5 to 5.

(3) The evaporation device according to (1) or (2), further comprising a heating medium-blowing pipe directly blowing a heating medium into the evaporator.

(4) A method for evaporation in which part of a liquid raw material that comprises two or more components and that is used in gas-phase catalytic reaction accompanied with heat generation is evaporated, the method comprising the step of supplying the liquid raw material to the evaporation device according to any one of (1) to (3).

(5) The method for evaporation according to (4), further comprising the step of directly blowing the components to be used in the gas-phase catalytic reaction or vapors thereof into the evaporator as the heating medium.

(6) The method for evaporation according to (5), wherein a ratio ($k_l/k_m$) is 1.5 or more, when a volatility of a component having the highest volatility among components which are not evaporated and have substantial concentrations is defined as $k_l$, and a volatility of a component having the lowest volatility among components which are evaporated and have substantial concentrations is defined as $k_m$, in the whole components summed up with the liquid raw material and the heating medium to be directly blown into the evaporator.

(7) The method for evaporation according to (5) or (6), wherein the liquid raw material comprising tertiary butyl alcohol or (meth)acrolein is used and water vapor is used as the heating medium.

(8) A method for producing methacrolein or (meth) acrylic acid, comprising the step of evaporating a liquid raw material comprising tertiary butyl alcohol or (meth)acrolein by the method for evaporation according to any one of (4) to (7).

## EFFECT OF THE INVENTION

According to the evaporation device and the method for evaporation of the present invention, part of a liquid raw material that contains two or more components and that is used in gas-phase catalytic reaction accompanied with heat generation can be stably evaporated.

The method for evaporation of the present invention is suitably used in a method for producing methacrolein or (meth)acrylic acid.

## BRIEF DESCRIPTION OF DRAWINGS

Figure 1: A schematic configuration diagram showing an embodiment of the evaporation section (multitubular heat exchanger method)

Figure 2: A schematic configuration diagram showing an embodiment of the evaporation section (outer jacket method)

Figure 3: A schematic configuration diagram showing an embodiment of the evaporation section (trace piping method)

Figure 4: A schematic configuration diagram showing an embodiment of the evaporation device (plate column method)

Figure 5: A schematic configuration diagram showing an embodiment of the evaporation device (packed column method)

EXPLANATION OF NUMERALS

11: Evaporation section
12: Evaporator
12a: Vapor take-out port
12b: Liquid discharging portion
13: Liquid discharge pipe
14: Multitubular heat exchanger
15: Piping
16: Piping
17: Pump
18: Heating medium supply pipe
19: Heating medium discharge pipe
21: Evaporation section
22: Outer jacket
23: Heating medium supply pipe
24: Heating medium discharge pipe
31: Evaporation section
32: Trace piping
33: Heating medium supply pipe
34: Heating medium discharge pipe
41: Evaporation device
42: Evaporation section
43: Plate column
44: Vapor lead-out pipe
45: Heating medium-blowing pipe
46: Liquid supply pipe
47: Liquid supply pipe
51: Evaporation device
52: Packed column
53: Liquid supply pipe

BEST MODE FOR CARRYING OUT THE INVENTION

< Evaporation device>

The evaporation device of the present invention is provided with an evaporation section which is equipped with an evaporator having a vapor take-out port at the top of the evaporator, a column section, the bottom of which is connected to the vapor take-out port of the evaporator, a liquid supply pipe connected to a middle or upper position of the column section, a vapor lead-out pipe connected to the top of the column section, and a liquid discharge pipe connected to the evaporator.

In such an evaporation device, when a liquid raw material is supplied from the liquid supply pipe, the liquid raw material is introduced into a middle or upper position of the column section, flows down inside the column section, and introduced into the evaporator from the bottom of the column section through the vapor take-out port. Then, in the evaporator, the heat quantity necessary for evaporating part of the liquid raw material is supplied to the liquid raw material with a heating mechanism through the heating medium and the liquid raw material is evaporated. Subsequently, the vapor inside the evaporator transfers through the vapor take-out port to the column section, rises upward in the column section, and is drawn out of the vapor lead-out pipe connected to the top of the column section and supplied to the reactor for gas-phase catalytic reaction. Further, the components left behind without being evaporated in the liquid raw material are discharged from the liquid discharge pipe to outside the evaporation device. Here, the evaporation section means a portion where the evaporator and the heating mechanism are combined, and does not include the column section.

The evaporation section is provided with the evaporator having the vapor take-out port at the top of the evaporator and the liquid discharging portion at the bottom of the evaporator, and the heating mechanism which supplies heat quantity to the liquid existing inside the evaporator through the heating medium. Further, the liquid discharge pipe is connected to the liquid discharging portion.

As the heating mechanism, the one that has a mechanism of heating the liquid raw material from inside or outside or both inside and outside the evaporator to evaporate the liquid raw material can be used. As such a heating mechanism of inside heating type or outside heating type, the same one as the one that is used in the conventional evaporator type can be used. As the outside heating type, a multitubular heat exchanger can be listed, and as the inside heating type, an outer jacket, trace piping, and the like can be listed. Among them, the multitubular heat exchanger is preferable because the quantity of the liquid raw material to be treated is large.

As the evaporation section that uses the multitubular heat exchanger as the heating mechanism, the one in which the evaporator and the heat exchanger are connected with the piping with which the liquid raw material is discharged from the liquid discharging portion and transferred to the heat exchanger and with the piping with which the liquid raw material and its vapor heated in the heat exchanger are introduced into the evaporator can be listed. As such an evaporation section, there are a thermosyphon type that does not have a pump between the heat exchanger and the evaporator and a forced circulation type that has a pump between the heat exchanger and the evaporator. The forced circulation type is preferable from the viewpoint of stability of the heat quantity to be supplied to the liquid raw material.

In addition to the above-mentioned heating mechanism, a heating medium-blowing pipe that directly blows the heating medium into the evaporator can be listed. When the component to be used in the gas-phase catalytic reaction or its vapor is used as the heating medium, the heating medium can be directly blown into the liquid raw material inside the evaporator by setting up the heating medium-blowing pipe.

These heating mechanisms can be used alone or in a combination of two or more kinds.

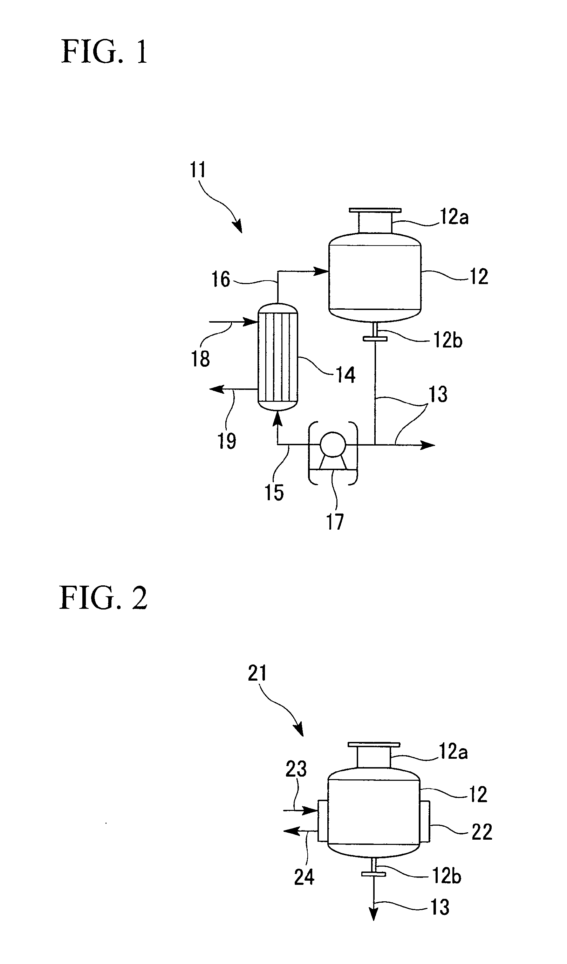

In Figures 1 to 3, each embodiment of the evaporation section, to which the liquid discharge pipe is connected, to be used in the present invention is shown, respectively.

Figure 1 is a schematic configuration diagram of evaporation section 11 which is equipped with a tubular heat exchanger. In the evaporation section 11, evaporator 12 has vapor take-out port 12a at the top and liquid discharging portion 12b at the bottom. Liquid discharge pipe 13 is connected to the liquid discharging portion 12b and the components which are not evaporated in the liquid raw material inside evaporator 12 can be discharged outside the system.

A heating mechanism equipped with multitubular heat exchanger 14 is installed on the evaporator 12 as the heating mechanism. The heating mechanism is composed of the heat exchanger 14, piping 15, one end of which is connected to the liquid discharge pipe 13 and the other end of which is connected to the inlet of the heat exchanger 14, piping 16, one end of which is connected to the outlet of the heat exchanger 14 and the other end of which is connected to the body of the evaporator 12, heating medium supply pipe 18 which supplies the heating medium, and heating medium discharge pipe 19 which discharges the heating medium.

Pump 17 is provided on the piping 15, by which the liquid raw material inside the evaporator 12 can be forced to circulate through the liquid discharge pipe 13, the piping 15, the heat exchanger 14, and the piping 16 to the evaporator 12. In the case that the evaporation section 11 is the thermosyphon type, the pump 17 may not be provided.

The heating medium supply pipe 18 and the heating medium discharge pipe 19 are respectively connected to the heat exchanger 14, by which the heat quantity necessary for evaporation can be supplied to the liquid raw material through the heating medium at the heat exchanger 14. A control valve to adjust the feed rate of the liquid raw material may be provided on the heating medium supply pipe 18.

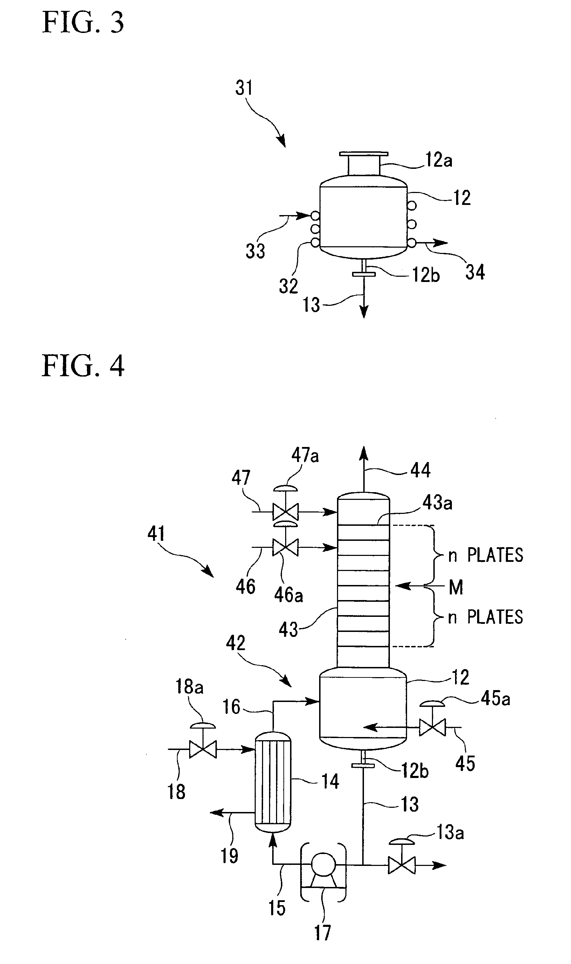

Figure 2 is a schematic configuration diagram of evaporation section 21 which is equipped with an outer jacket as the heating mechanism. In Figures 2 and 3, the same numerals as those of Figure 1 are attached to the constitutional elements corresponding to the constitutional elements shown in Figure 1, and the explanations thereof are omitted.

The evaporation section 21 is composed of, as the heating mechanism, outer jacket 22, heating medium supply pipe 23 which supplies the heating medium to the outer jacket 22, and heating medium discharge pipe 24 which discharges the heating medium inside the outer jacket 22.

The outer jacket 22 is installed on the outside of the evaporator 12, by which the evaporator 12 is heated through the heating medium and the heat quantity necessary for evaporation can be supplied to the liquid raw material contained in the evaporator 12.

Figure 3 is a schematic configuration diagram of evaporation section 31 which is equipped with a trace piping as the heating mechanism. The evaporation section 31 is composed of, as the heating mechanism, trace piping 32, heating medium supply pipe 33 which supplies the heating medium to the trace piping 32, and heating medium discharge pipe 34 which discharges the heating medium inside the trace piping 32.

The trace piping 32 is installed on the outside of the evaporator 12, by which the evaporator 12 is heated through the heating medium and the heat quantity necessary for evaporation can be supplied to the liquid raw material.

The column section, the bottom of which is connected to the vapor take-out port 12a of the evaporator 12, is composed of at least one of a plate column and a packed column. A column containing plates is called as the plate column and a column containing packings is called as the packed column. The column section may contain both of the plates and the packings.

As the plate column and the packed column, the ones with the same configurations as those generally used in refining, distillation, or the like are available.

As the plate to be used in the plate column, a sieve tray with a weir, a sieve tray without a weir, a turbogrid plate, or the like can be listed. These trays may be properly selected in accordance with the properties of the liquid raw material to be treated. For example, when the liquid raw material contains an easily polymerizable material, the sieve tray without a weir is preferable. The easily polymerizable material means a compound having a polymerizable functional group (for example, vinyl group).

As the packings to be used in the packed column, irregular packings or regular packings to be used in an absorption column or distillation column can be listed. These packings may be properly selected in accordance with the properties of the liquid raw material to be handled. For example, when the liquid raw material is an easily polymerizable material, the regular packings are suitable.

The column section preferably has a structure in which the liquid holdup inside the column section is small. As the column section having such a structure, a column section which is composed of a plate column having a plate number of 1 to 10 or a packed column having a packed length equivalent to theoretical plate number of 0.5 to 5 can be listed.

Here, "the packed length in the packed column" is a height of the packings being packed inside the column (a filling part), and in the case that there are a plurality of filling parts, it is a sum of the height of each filling part.

The upper limit of the plate number of the plate column or the upper limit of the theoretical plate number of the packed column is selected from the viewpoint that the pressure inside the evaporator should not be unnecessarily raised or the equipment cost should be suppressed. The plate number of the plate column is preferably 3 to 7. The theoretical plate number of the packed column is preferably 1.5 to 3.5.

In the present invention, the liquid supply pipe that supplies the liquid raw material is connected to a middle or upper position of the column section. Here, "the middle position of the column section" is the portion where the plate or the filling part is provided, and in the case of the plate column, it is from the lower edge of the plate that is set at the lowermost side to the upper edge of the plate that is set at the uppermost side, and in the case of the packed column, it is from the lower edge of the filling part that is arranged at the lowermost side to the upper edge of the filling part that is arranged at the uppermost side. Further, "the middle or upper position of the column section" is the position between the lower edge of the middle position and the top of the column section.

The position where the liquid supply pipe is connected is preferably a position between the upper edge of the middle position and the top of the column section (upper position). The number of the liquid supply pipe may be one or two or more. When the two or more liquid supply pipes are provided, components to be used in gas-phase catalytic reaction accompanied with heat generation can be supplied from each liquid supply pipe to the column section. A control valve to adjust the feed rate of a liquid may be provided on the liquid supply pipe.

The vapor lead-out pipe that leads out the vapor inside the column section is connected to the top of the column section. The other end of the vapor lead-out pipe is connected to the reactor for gas-phase catalytic reaction and the vapor inside the column section can be supplied to the reactor.

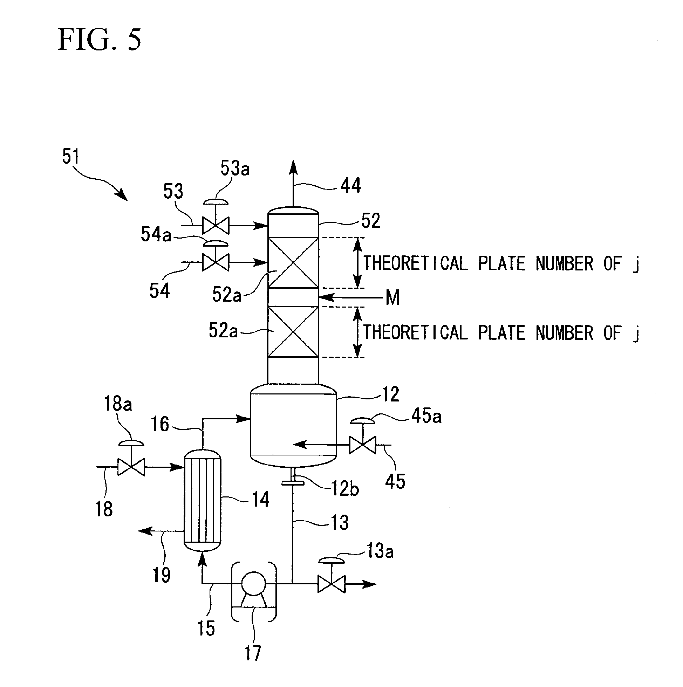

The preferable embodiments of the evaporation device of the present invention are shown in Figures 4 and 5. In Figures 4 and 5, the same numerals as those of Figure 1 are attached to the constitutional elements corresponding to the constitutional elements shown in Figure 1, and the explanations thereof are omitted.

Evaporation device 41 shown in Figure 4 is equipped with evaporation section 42 and plate column 43 which constitutes a column section, and vapor lead-out pipe 44 is connected to the top of the plate column 43. The evaporation section 42 has the constitution of the evaporation section 11 shown in Figure 1 and further, as a heating mechanism, has the constitution of the heating medium-blowing pipe 45, through which the heating medium is blown into the evaporator 12. Further, in the evaporation section 42, control valve 13a to adjust the discharge quantity of the liquid raw material, control valve 18a to adjust the feed rate of the heating medium, and control valve 45a to adjust the quantity of blowing the heating medium into the evaporator are provided on the liquid discharge pipe 13, on the heating medium supply pipe 18, and on the heating medium-blowing pipe 45, respectively.

The plate column 43 is installed on the vapor take-out port (not shown in the figure) of the evaporator 12. A plurality of plates (trays) 43a are provided inside the plate column 43. Liquid supply pipes 46 and 47 are connected at the middle position of the plate column 43 (position marked as M in Figure 4) and the upper position, respectively, and control valves 46a and 47a to adjust the feed rate of the liquid raw material are provided on these pipes, respectively.

Evaporation device 51 shown in Figure 5 is different from the evaporation device 41 in that the column section is constituted of packed column 52. Two filling parts 52a are provided in the packed column 52. Liquid supply pipes 53 and 54 are connected at positions upper than the middle position of the packed column 52 (position marked as M in Figure 5), and control valves 53a and 54a to adjust the feed rate of the liquid raw material are provided on the liquid supply pipes 53 and 54, respectively.

In the case that necessary aids (polymerization inhibitor, defoaming agent, and the like) are used depending on the properties of a liquid raw material to be handled when part of the liquid raw material that comprises two or more components and that is used in gas-phase catalytic reaction accompanied with heat generation is evaporated using the evaporation device of the present invention, it is possible to provide a supply piping for the aids at a proper position of the evaporation device of the present invention. Further, in the evaporation device of the present invention, incidental facilities such as apparatuses of various measurements and controls may be provided at an optional position such as evaporation section, column section, liquid supply pipe, or vapor lead-out pipe.

In general, in the case of evaporating a liquid raw material containing two or more components, there is a case that an evaporation operation is carried out in such a way that the greater part of the liquid raw material is evaporated and the remaining part of the liquid raw material that is a high boiling point material is not evaporated. Such an evaporation operation does not need a precise distillation separation operation and can be adequately carried out by a separation with what is called simple distillation using an evaporator. However, variation in a composition or a feed rate of a feed gas in a short time cycle cannot be allowed in gas-phase catalytic reaction, as mentioned above. When an evaporation operation is carried out using a conventional evaporator type evaporation device, in which part of the liquid raw material that contains two or more components and that is used in gas-phase catalytic reaction is evaporated and another part is not evaporated, it is difficult to control the variation in the vapor composition or the amount of evaporation in a short time cycle because the system is susceptible to disturbance.

On the other hand, the evaporation device of the present invention can stably carry out evaporation of a liquid raw material that contains two or more components and that is used in gas-phase catalytic reaction accompanied with heat generation because a column section is provided on a evaporator and a liquid supply pipe is connected to a specified position of the column section. As the reason for this, it is presumed that the liquid raw material flowing down inside the column section and the vapor going up inside the column section come into contact with each other and this serve as a function of distillation when part of the liquid raw material is evaporated using the evaporation device of the present invention, and hence the vapor composition and the liquid composition get near, so that the variation in the vapor composition be eased.

Consequently, the evaporation device of the present invention is useful as the evaporation device which evaporates a liquid raw material that contains two or more components and that is used in gas-phase catalytic reaction, and in particular, it is preferable in the case that the liquid raw material having a ratio ($k_l/k_m$) of 1.5 or more and the heating medium to be blown into the evaporator are used, when a volatility of a component having the highest volatility among components which are not evaporated (non-evaporating components) and have substantial concentrations is defined as $k_l$, and a volatility of a component having the lowest volatility among components which are evaporated (evaporating components) and have substantial concentrations is defined as $k_m$, in the composition of the whole components summed up with the liquid raw material and the heating medium to be blown into the evaporator.

Here, "a component has substantial concentration" means that the component is one of the evaporating components or non-evaporating components, the concentration of which has to be controlled and is at least above the analytical detection limit. The component having the lowest volatility among the evaporating components which have substantial concentrations or the component having the highest volatility among the non-evaporating components which have substantial concentrations is called key component.

<Method for evaporation>

The method for evaporation of the present invention is a method for evaporating part of the liquid raw material and has a step of supplying the liquid raw material to the evaporation device of the present invention. The liquid raw material is supplied from the liquid supply pipe to the evaporation device of the present invention and firstly introduced into the middle or upper position of the column section, flowed down inside the column section, and introduced into the evaporator from the bottom of the column section through the vapor take-out port. Subsequently, in the evaporation section, the liquid raw material is evaporated with the heating mechanism, the heat quantity necessary for evaporating part of the liquid raw material being supplied to the liquid raw material through the heating medium. Then, the vapor inside the evaporator is transferred to the column section through the vapor take-out port, raised up inside the column section, drawn out of the vapor lead-out pipe connected to the top of the column section, and supplied to the reactor for gas-phase catalytic reaction. Further, the components left behind without being evaporated in the liquid raw material are discharged from the liquid discharge pipe to outside the evaporation device.

In the method for evaporation of the present invention, it is preferable to use the evaporator equipped with a heating medium-blowing pipe which directly blows the heating medium into the evaporator and to carry out the step of blowing the components to be used in the gas-phase catalytic reaction or vapors thereof as the heating medium from the heating medium-blowing pipe into the evaporator. In gas-phase catalytic reaction accompanied with heat generation, there are many cases where water is added to a feed gas as a diluent to suppress temperature rise of a catalyst layer in a reactor or to improve catalyst selectivity or life. In these cases, it is useful to use water vapor as the heating medium and to blow necessary quantity of water or water vapor as the diluent directly into the liquid raw material inside the evaporator.

At this time, as the heating mechanism of the evaporation section, the heating medium-blowing pipe may be used alone or in combination with another heating mechanism (multitubular heat exchanger, outer jacket, trace piping, or the like). When the heat quantity necessary for evaporation is larger than the quantity of the water or water vapor to be blown into, it is preferable to jointly use the other heating mechanism, in particular, the heat exchanger.

The method for evaporation of the present invention is particularly preferable, as mentioned above, in the case that the liquid raw material having a ratio ($k_l/k_m$) of 1.5 or more and the heating medium to be blown into the evaporator are evaporated, when a volatility of a component having the highest volatility among components which are not evaporated (non-evaporating components) and have substantial concentrations is defined as $k_l$, and a volatility of a component having the lowest volatility among components which are evaporated (evaporating components) and have substantial concentrations is defined as $k_m$, in the composition of the whole components summed up with the liquid raw material and the heating medium to be blown into the evaporator. Here, "have substantial concentrations" means what has been mentioned above.

In the composition of the whole components summed up with the liquid raw material composed of n components and the heating medium to be blown into the evaporator, when each of the n components is defined in the descending order of volatility as the first component, the second component, ....., the nth component, volatility $k_i$ of the ith component (i is an integer of from 1 to n) which composes the liquid raw material and the heating medium to be blown into the evaporator is defined by the following equation (1).

$$k_i = p_i/x_i \cdots (1)$$

In the equation (1), $x_i$ represents molar fraction of i component in the liquid inside the evaporation device, and $p_i$ represents partial pressure of i component in a gas that is in equilibrium with $x_i$.

When from the first component to the mth component in the n components (m is an integer from l to n, and a component having a substantial concentration) are evaporating components that are used in the gas-phase catalytic reaction after being evaporated, and from the lth component to the nth component (l is an integer from l to n, and a component having a substantial concentration) are non-evaporating components, the component having the lowest volatility among the evaporating components is the mth component and its volatility is $k_m$. Further, the component having the highest volatility among the non-evaporating components is the lth component and its volatility is $k_l$. The method for evaporation of the present invention is preferably used in the case that the liquid raw material having the ratio of the $k_l$ to the $k_m$, that is ($k_l/k_m$), of 1.5 or more and the heating medium to be blown into the evaporator are evaporated.

In the case that the liquid raw material and the heating medium to be blown into the evaporator having both key components (the mth component and the lth component) with such separated volatilities are evaporated, evaporation with what is called simple distillation using a conventional evaporator type causes a great difference between the liquid composition and the vapor composition in the evaporator, and causes a great variation in the evaporation composition with some disturbance. However, in the present invention, the vapor composition and the liquid composition get near owing to the distillation effect inside the column section by using the evaporation device of the present invention, and hence the variation in the composition caused by disturbance is eased.

The method for evaporation of the present invention is preferably used in evaporation of the liquid raw material having the ratio ($k_l/k_m$) of 2 or more. The volatility of each component is obtained by the foregoing equation (1).

As a typical example of the gas-phase catalytic reaction accompanied with heat generation which uses the liquid raw material evaporated by the method of the present invention, gas-phase catalytic reaction (gas-phase catalytic oxidation reaction) between tertiary butyl alcohol or (meth)acrolein and oxygen can be listed. Methacrolein or methacrylic acid is produced by the gas-phase catalytic oxidation reaction of tertiary butyl alcohol. Further, methacrylic acid is produced by the gas-phase catalytic oxidation reaction of methacrolein. Further, acrylic acid is produced by the gas-phase catalytic oxidation reaction of acrolein. The method for evaporation of the present invention is suitable, in particular, in the case that the liquid raw material containing tertiary butyl alcohol or (meth)acrolein and water vapor as the heating medium are used.

The typical example of the feed gas composition of the gas-phase catalytic oxidation reaction of tertiary butyl alcohol is 5% of tertiary butyl alcohol, 10% of oxygen, 5% of water, and the remainder, inert gas such as nitrogen. In other words, water does not directly participate in the gas-phase catalytic oxidation reaction of tertiary butyl alcohol, however, it has the effect of diluting the reactant gas or the like, and hence it is one of the necessary components in the feed gas.

While, tertiary butyl alcohol is generally synthesized by hydration reaction from one of the products of naphtha cracking, that is, an isobutene-containing fraction having 4 carbon atoms or from an isobutene-containing by-product gas having 4 carbon atoms from fluid catalytic cracking apparatus, and the products include, in addition to tertiary butyl alcohol, water and impurities such as multimers of isobutene and secondary butyl alcohol.

Because water is a necessary component in the feed gas of the gas-phase catalytic oxidation reaction of tertiary butyl alcohol, as stated above, tertiary butyl alcohol synthesized as mentioned above is usually used directly without carrying out separation of water as the liquid raw material to be used for the gas-phase catalytic oxidation reaction of tertiary butyl alcohol. In other words, the liquid raw material to be used for the gas-phase catalytic oxidation reaction of tertiary butyl alcohol usually contains tertiary butyl alcohol, water, multimers of isobutene, secondary butyl alcohol, and the like. Impurities such as multimers of isobutene, secondary butyl alcohol, and high boiling point compounds originated from the catalyst of hydration reaction are included in the liquid raw material, though they are at low concentrations. These impurities are not preferable for the catalysts or products qualities of the gas-phase catalytic oxidation reaction, and they are preferably discharged from the evaporation device without being evaporated at the evaporation device.

The content ratio of tertiary butyl alcohol to water in the liquid raw material is variable depending on a production process of tertiary butyl alcohol. When water content in the liquid raw material is large as compared with the target feed gas composition suitable for the gas-phase catalytic oxidation reaction of tertiary butyl alcohol, part of water is separated without being evaporated. On the other hand, when water content in the liquid raw material is small, water may be added to the liquid raw material at the evaporation device, or water vapor may be directly blown into the evaporator as the heating medium from the heating medium-blowing pipe using evaporation device provided with the heating medium-blowing pipe.

In this example, the component having substantial concentration and having the lowest volatility among the evaporating components is water, and the component having substantial concentration and having the highest volatility among the non-evaporating components is tertiary butyl alcohol. Further, the relative volatility in the composition summed up with the quantities of the liquid raw material and water vapor to be blown into the evaporator is about 2.5, and variation in the vapor composition caused by disturbance is large in the case of a conventional evaporation device, however, such a liquid raw material can be stably evaporated according to the present invention.

In the case of synthesizing (meth)acrylic acid by the gas-phase catalytic oxidation reaction of (meth) acrolein, a stable evaporation can be carried out using the method for evaporation of the present invention as shown below.

The typical example of the feed gas composition of the gas-phase catalytic oxidation reaction of (meth)acrolein is 5% of (meth)acrolein, 10% of oxygen, and 10% of water. Water does not directly participate in the gas-phase catalytic oxidation reaction of (meth)acrolein, however, it has the effect of diluting the reactant gas or the like, and hence it is one of the necessary components in the feed gas. While, (meth)acrolein can be produced by the gas-phase catalytic oxidation reaction of at least one compound of tertiary butyl alcohol, isobutene, and propylene with oxygen, and the typical composition of the reaction products is 4% of low boiling point compounds such as acetone, 86% of (meth)acrolein, and 10% of water (all of which is in mol%).

Water produced as a by-product in the reaction for synthesizing (meth)acrolein mentioned above is usually used directly as the liquid raw material for the gas-phase catalytic oxidation reaction of (meth)acrolein because water is a necessary component in the feed gas of the gas-phase catalytic oxidation reaction of (meth) acrolein, as stated above. Further, inhibitors and polymerized compounds are contained in (meth)acrolein, though they are at low concentrations. In other words, the liquid raw material for the gas-phase catalytic oxidation reaction of (meth)acrolein usually contains, in addition to the low boiling compounds such as (meth)acrolein, water, and acetone, the high boiling compounds such as inhibitors and polymerized compounds, though they are at low concentrations, and they are preferably discharged from the evaporation device without being evaporated at the evaporation device. By doing so, stable evaporation can be carried out, and moreover, there are merits in which concentration of (meth)acrolein in the evaporator becomes low as compared with the case of conventionally used evaporator type evaporation device and hence loss of the main raw material is avoided, and generation of the polymerized compounds at the evaporating section is suppressed and hence safety operation can be carried out.

The compositional ratio of water to (meth)acrolein in the foregoing liquid raw material is low as compared with that in the vapor to be supplied to the oxidation reactor. Consequently, at least one component of water and water vapor is added to the liquid raw material using the evaporation device of the present invention at the time of evaporating the liquid raw material containing (meth)acrolein, and the compositional ratio of water to (meth)acrolein in the vapor to be supplied to the oxidation reactor can be easily raised.

In this example, the component having substantial concentration and having the lowest volatility among the evaporating components is water, and the component having substantial concentration and having the highest volatility among the non-evaporating components is (meth)acrolein. Further, the relative volatility in the composition summed up with the quantities of the liquid raw material and water vapor to be blown into the evaporator is about 4, and variation in the vapor composition is large in the case of a conventional evaporation device, however, such a liquid raw material can be stably evaporated according to the present invention.

EXAMPLES

Hereinafter, the present invention will be explained in more detail by examples, however, the present invention is not limited to the following examples.

Example 1

In the present example, in the process for synthesizing (meth)acrolein by gas-phase catalytic oxidation reaction of tertiary butyl alcohol with oxygen, the feed gas to be supplied to the reactor to be used for carrying out the reaction was produced using the evaporation device 41 having the constitution shown in Figure 4.

In the evaporation device 41, a vertical multitubular heat exchanger was used as the heat exchanger 14, and heating was carried out using a thermosyphon type (pump 17 not being used) and water vapor having the pressure of 0.3 MPa as the heating medium. As the plate column 43, the one equipped with 5 sieve trays without weirs was used.

The composition of the liquid raw material to be used for the gas-phase catalytic oxidation reaction mentioned above was 60 mol% of tertiary butyl alcohol, 39.9 mol% of water, and 0.1 mol% of secondary butyl alcohol, all of which were high boiling point compounds.

The component having substantial concentration and having the lowest volatility among the evaporating components is water, and the component having substantial concentration and having the highest volatility among the non-evaporating components is tertiary butyl alcohol. Further, the relative volatility in the composition summed up with the quantities of the liquid raw material and water vapor to be blown into the evaporator is about 2.5.

The aforementioned liquid raw material was supplied to the liquid supply pipe 47 (on the uppermost tray) of the evaporation device 41.

Twenty mol% of water vapor with respect to the liquid raw material was directly blown into the evaporator 12 through the heating medium-blowing pipe 45, and at the same time, the liquid raw material was heated and evaporated at the heat exchanger 14 and the resultant vapor was drawn out of the top of the plate column 43 through the vapor lead-out pipe 44 and supplied to the reactor.

In the supplied liquid raw material, 0.01 mol% of tertiary butyl alcohol and 2 mol% of water were discharged from the liquid discharge pipe 13 without being evaporated.

The feed rate of the liquid raw material, the discharged quantity of the non-evaporating components in the liquid raw material, and the quantity of the water vapor to be blown into the evaporator 12 were controlled within the error of $\pm$ 0.5% by a computer controlled system of flow rate controllers provided in the evaporation device 41. The preset value for the control of the flow rate of the water vapor was set adopting a control system which slowly changes the preset value using cascade control by the liquid surface in the evaporator 12.

The reactor to which the liquid raw material for the oxidation reaction was supplied was a multitubular reactor using steel tubes (reaction tubes) having inside diameter of 25 mm and length of 5 m, and a mechanism of circulating niter outside the reaction tubes was provided to remove the heat of reaction. A publicly known molybdenum-cobalt-nickel oxide catalyst was used for the catalyst layer located at the center part of the reactor. A thermocouple was provided in the reactor so that the temperature distribution along the direction of the feed gas flow can be measured.

The composition (composition at the inlet of the reactor) of the vapor supplied from the evaporation device (feed gas) was 5 mol% of tertiary butyl alcohol, 10 mol% of oxygen, 5 mol% of water, and the remainder such as other inert gas components. The gas temperature and the pressure at the entrance of the catalyst layer were 295°C and 0.14Mpa, respectively.

Under this condition, the vapor from the evaporation device 41 was supplied to the reactor and the gas-phase catalytic oxidation reaction was carried out and methacrolein was synthesized.

As a result, there appeared no fluctuation in the differences between the maximum temperatures of the catalyst layers of respective reaction tubes and the temperature of the niter during a short time and a very stable state could be maintained. Further, the differences in temperature distribution among 100 reaction tubes of which the temperature distributions were measured were small with the average value of the differences between the maximum temperatures of the catalyst layers and the temperature of the niter being 30°C and the fluctuation thereof being $\pm$ 2°C, and an excellent state was maintained.

Comparative Example 1

The same procedure as in Example 1 was carried out to synthesize methacrolein using the same evaporation device 41 as in Example 1 except that the plate column 41 was not equipped, and the liquid supply pipe 47 was connected directly to the body of the evaporator 12, and the vapor lead-out pipe 44 is connected to the vapor take-out port of the evaporator 12.

As a result, the differences between the maximum temperatures of the catalyst layers of respective reaction tubes and the temperature of the niter during a short time were as large as 30 $\pm$ 7°C and a stable state could not be maintained. Further, in 5 reaction tubes out of 100 reaction tubes of which the temperature distributions were measured, the differences between the maximum temperatures of the catalyst layers of respective reaction tubes and the temperature of the niter became 45°C or more and what is called runaway reaction states were raised and the catalysts were deactivated.

INDUSTRIAL APPLICABILITY

According to the present invention, part of a liquid raw material that contains two or more components and that is used in gas-phase catalytic reaction accompanied with heat generation can be stably evaporated and hence variation in a feed rate or a gas composition of a feed gas in a short cycle can be suppressed.

## Claims

1. An evaporation device that evaporates part of a liquid raw material that comprises two or more components and that is used in gas-phase catalytic reaction accompanied with heat generation and supplies the part of the liquid raw material to a reactor for the gas-phase catalytic reaction accompanied with heat generation, comprising:

    an evaporation section that is equipped with an evaporator having a vapor take-out port at the top and evaporates the liquid raw material by heating the liquid raw material from inside or outside or both inside and outside the evaporator;
    a column section composed of at least one of a plate column and a column containing packings, the bottom of which is connected to the vapor take-out port;
    a liquid supply pipe supplying the liquid raw material to a middle or upper position of the column section;
    a vapor lead-out pipe leading out vapor from the top of the column section and supplying the vapor to the reactor for the gas-phase catalytic reaction; and
a liquid discharge pipe discharging the liquid raw material in the evaporator.

2. The evaporation device according to claim 1, wherein the column section is composed of a plate column having a plate number of 1 to 10 or a packed column having a packed length equivalent to theoretical plate number of 0.5 to 5.

3. The evaporation device according to claim 1 or 2, further comprising a heating medium-blowing pipe directly blowing a heating medium into the evaporator.

4. A method for evaporation in which part of a liquid raw material that comprises two or more components and that is used in gas-phase catalytic reaction accompanied with heat generation is evaporated, the method comprising the step of supplying the liquid raw material to the evaporation device according to any one of claims 1 to 3.

5. The method for evaporation according to claim 4, further comprising the step of directly blowing the components to be used in the gas-phase catalytic reaction or vapors thereof into the evaporator as the heating medium.

6. The method for evaporation according to claim 5, wherein a ratio ($k_l/k_m$) is 1.5 or more, when a volatility of a component having the highest volatility among components which are not evaporated and have substantial concentrations is defined as $k_l$, and a volatility of a component having the lowest volatility among components which are evaporated and have substantial concentrations is defined as $k_m$, in the whole components summed up with the liquid raw material and the heating medium to be directly blown into the evaporator.

7. The method for evaporation according to claim 5 or 6, wherein the liquid raw material comprising tertiary butyl alcohol or (meth)acrolein is used and water vapor is used as the heating medium.

8. A method for producing methacrolein or (meth)acrylic acid, comprising the step of evaporating a liquid raw material comprising tertiary butyl alcohol or (meth)acrolein by the method for evaporation according to any one of claims 4 to 7.

FIG. 1

FIG. 2

FIG. 3

FIG. 4

FIG. 5

THEORETICAL PLATE NUMBER OF j

M

THEORETICAL PLATE NUMBER OF j

<table>
<tr><td colspan="2" align="center">**INTERNATIONAL SEARCH REPORT**</td><td>International application No.<br>PCT/JP2008/058925</td></tr>
</table>

**A. CLASSIFICATION OF SUBJECT MATTER**
*B01D1/14*(2006.01)i, *C07C45/38*(2006.01)i, *C07C47/22*(2006.01)i, *C07C51/235*
(2006.01)i, *C07C57/055*(2006.01)i

According to International Patent Classification (IPC) or to both national classification and IPC

**B. FIELDS SEARCHED**

Minimum documentation searched (classification system followed by classification symbols)
B01B1/00, B01D1/00-5/00, C07B33/00, C07C45/00, C07C47/00, C07C51/00,
C07C57/00

Documentation searched other than minimum documentation to the extent that such documents are included in the fields searched
Jitsuyo Shinan Koho        1922-1996    Jitsuyo Shinan Toroku Koho    1996-2008
Kokai Jitsuyo Shinan Koho    1971-2008    Toroku Jitsuyo Shinan Koho    1994-2008

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 8-71432 A (Phillips Petroleum Co.),<br>19 March, 1996 (19.03.96),<br>Figs. 1, 2 | 1-5<br>6-8 |
| X<br>A | JP 2004-267816 A (Sasakura Engineering Co.,<br>Ltd.),<br>30 September, 2004 (30.09.04),<br>Full text; all drawings | 1-6<br>7,8 |
| X | JP 2002-316804 A (Sumitomo Chemical Co., Ltd.),<br>31 October, 2002 (31.10.02),<br>Fig. 1; Par. Nos. [0011] to [0018] | 1,2 |
| X | JP 7-39745 A (Kabushiki Kaisha Seta Giken),<br>10 February, 1995 (10.02.95),<br>Full text; Fig. 3 | 1,2 |

| ☒ Further documents are listed in the continuation of Box C. | ☒ See patent family annex. |
|---|---|

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>19 August, 2008 (19.08.08) | Date of mailing of the international search report<br>02 September, 2008 (02.09.08) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2008/058925

C (Continuation). DOCUMENTS CONSIDERED TO BE RELEVANT

| Category* | Citation of document, with indication, where appropriate, of the relevant passages | Relevant to claim No. |
|---|---|---|
| A | JP 50-108208 A (Nippon Shokubai Kagaku Kogyo Co., Ltd.), 26 August, 1975 (26.08.75), Full text; all drawings | 7,8 |

Form PCT/ISA/210 (continuation of second sheet) (April 2007)

<table>
<tr><td colspan="2"><strong>INTERNATIONAL SEARCH REPORT</strong><br>Information on patent family members</td><td>International application No.<br><br>PCT/JP2008/058925</td></tr>
</table>

| | | |
|---|---|---|
| JP 8-71432 A | 1996.03.19 | US 5527980 A |
| | | US 5547909 A |
| | | EP 703002 A1 |
| | | EP 669301 A1 |
| | | DE 69513118 D |
| | | DE 69513118 T |
| | | DE 69505710 C |
| | | NO 953379 A |
| | | AU 2321795 A |
| | | FI 954054 A |
| | | BR 9503035 A |
| | | CA 2152337 A |
| | | HU 75950 A |
| | | AT 186231 T |
| | | ES 2137425 T |
| | | DK 703002 T |
| | | GR 3032305 T |
| | | ES 2123168 T |
| | | AT 172952 T |
| | | DK 669301 T |
| | | FI 954054 A0 |
| JP 2004-267816 A | 2004.09.30 | (Family: none) |
| JP 2002-316804 A | 2002.10.31 | (Family: none) |
| JP 7-39745 A | 1995.02.10 | (Family: none) |
| JP 50-108208 A | 1975.08.26 | (Family: none) |

Form PCT/ISA/210 (patent family annex) (April 2007)

| INTERNATIONAL SEARCH REPORT | International application No. |
|---|---|
| | PCT/JP2008/058925 |

It is described in claim 1 that "an evaporation apparatus evaporating a portion of a liquid material containing two or more types of components for use in exothermic vapor-phase catalytic reaction and feeding the same to a reactor for the vapor-phase catalytic reaction." It appears that this description specifies the evaporator as an article in dependence on the destination of connection thereof. However, even if technical common knowledge is taken into account, the technical matter as the invention of apparatus seemingly limited by the destination of connection is ambiguous.

Therefore, in the judgment concerning the novelty and inventive step in this search, the afore-said specifying in dependence on the destination of connection of the evaporator is not considered with respect to claim 1, and claims 2 and 3 referring to the claim.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007129038 A

**Non-patent literature cited in the description**

- Outline of Process Design. *Chemical Engineering Society,* August 1973, 63-72